# EUROPEAN PATENT SPECIFICATION

(11) **EP 1 919 359 B1**
(45) Date of publication and mention of the grant of the patent: **25.04.2018**
(21) Application number: 06744807.6
(22) Date of filing: 15.05.2006
(51) Int. Cl.: A61B 5/11

(54) **ILLUMINATION SYSTEM**
BELEUCHTUNGSSYSTEM
SYSTEME D'ECLAIRAGE

(30) Priority: 26.07.2005 JP 2005215498; 26.07.2005 JP 2005215512; 21.11.2005 JP 2005335596
(43) Date of publication of application: 14.05.2008
(73) Proprietor: Panasonic Intellectual Property Management Co., Ltd., Osaka-shi, Osaka 540-6207 (JP)
(72) Inventor: NOGUCHI, Hiroki c/o Matsushita Electr.Works, Ltd., Kadoma-shi Osaka 571-8686 (JP); INOUE, Manabu c/o Matsushita Electric Works, Ltd., Kadoma-shi Osaka 571-8686 (JP); TODA, Naohiro c/o Matsushita Electric Works, Ltd., Kadoma-shi Osaka 571-8686 (JP)
(74) Representative: Samson & Partner Patentanwälte mbB
(86) International application number: PCT/IB2006/001440
(87) International publication number: WO 2007/012927

(56) References cited:
- EP-A- 1 209 411
- US-A1- 2003 095 476
- US-A1- 2003 231 495
- US-A1- 2004 049 132
- US-A1- 2005 143 617
- US-B1- 6 547 728

## Description

### Technical Field

The present invention relates to an illumination system; and, more particularly, to an illumination system capable of evaluating and/or improving its user's sleep quality.

### Background Art

US 2005/014367 A1 discloses a sleep system used for analysis and modification of sleep. The sleep system includes a processor, a network connection, a set of body and sleep monitoring sensors, environmental monitoring sensors, an environmental control interface, local storage and memory. The document also discloses that a dim light is provided during sleep to counteract wakeups or other undesirable sleeping pattern or behaviour.

US-B1-6 548 728 refers to a device for measuring organism condition. The document discloses a device incorporated in a watch designed to awaken the user at the end of a sleep cycle. The casing of the watch includes an electronic circuit of filtration, that receives electric signals from sensor and buttons and emits electric signals to be received by the display, the electro-acoustic transducers and the vibrator.

US 2003/0095476 A1 refers to a method and apparatus for a waking system that wakes an individual gradually over a period of time in order to promote the wellness of that individual. The waking system is made up of a number of distinct subsystems and components, each of which interacts with the controller which also regulates the light source.

US 2004/049132 A1 discloses a device for monitoring body activity, the device comprising an actimetry sensor for measuring body activity, storage means for receiving data from the actimetry sensor, means for analysing the data to provide advisory information, and means for displaying the advisory information to a user. The advisory information provided to the user may include an indication of the quality of the activity, such as the quality of the sleep.

EP-A-1 209 411 refers to a night light device, in particular for children, which gradually reduces the light intensity of the illuminant.

US 2003/0231495 A1 discloses a nightlight for phototherapy which produces light at various preselected intensities and wavelengths which are ideally suited for promoting physiological and therapeutic effects on the human body.

There have been developed several techniques for investigating the user's bodily state from going-to-bed to waking-up to determine whether the user fell asleep and/or evaluate a quality of the users' sleep (see, for example, Japanese Patent Laid-Open Application No. H5-245148; Japanese Patent Laid-Open Application No. H5-337103; Japanese Patent Laid-Open Application No. H5-095934; Japanese Patent Laid-Open Application No. H5-095935; Japanese Patent Laid-Open Application No. H5-111474; Japanese Patent Laid-Open Application No. H6-098863; and Japanese Patent Laid-Open Application No. 2000-316832).

Further, it is known that light suppresses secretion of melatonin, which is a sleep hormone secreted from a part of brain during night time (see, for example, Lewy A. J., Wehr T. A., Goodwin F. K., Newsome D.A. and Markey S.P., "Light Suppresses Melatonin Secretion in Humans", Science 1980 Dec. 12; 210(4475): 1267-1269), and illumination devices for reducing the melatonin suppression have been developed (see, for example, Japanese Patent Application No. 2005-074796). In addition, there is also known a device for controlling an illuminance of a lamp to be attenuated with time by using a timer (see, for example, Japanese Patent Laid-open Application No. 2002-200171).

In accordance with the above-mentioned techniques for investigating the user's bodily state from going-to-bed to waking-up, a body movement of the user is measured using, for example, a piezo-electric sensor. Based on the measurement, a point in time at which the body movement measured is greater than a specific magnitude for the first time is determined as "going-to-bed," and a point in time at which it is smaller than another specific magnitude is determined as "sleep onset."

The information about the bodily state from going-to-bed to sleep onset can possibly be used in the following two applications. Firstly, it can be used for controlling an illumination for reducing the melatonin suppression. Since melatonin begins to be secreted at relatively deep into the night, it is necessary to convert a normal illumination to an illumination for reducing melatonin suppression (hereinafter, abbreviated as "melatonin illumination") at a certain point in time during the night. Specifically, it is known that the melatonin secretion becomes noticeable about two hours prior to bed time or 14 hours after waking up (see, for example, Burgess H. J., Savic N., Sletten T., Roach G., Gilbert S. S., Dawson D., "The Relationship Between the Dim Light Melatonin Onset and Sleep on a Regular Schedule in Young Healthy Adults", Behavioral Sleep Medicine 2003, Vol. 1, No. 2, pp. 102-114), and it is preferable convert the normal illumination to the melatonin illumination based on the above-mentioned result. The process described above, however, would prove inconvenient for the user if the user is required to take into consideration his/her sleeping behavior and is required to perform a manual conversion from the normal illumination to the melatonin illumination. Therefore, it is necessary to provide an apparatus capable of automatically detecting the user's sleeping and wake-up time to automatically perform the above-mentioned conversion of illumination, with a view to providing the user with an optimal lighting environment for sleeping without the user ever becoming conscious of the conversion.

Secondly, the information about the bodily state from going-onto-bed to sleep onset can also be used for a sleep evaluation. One factor for evaluating the user's quality of sleep is how rapidly he/she falls asleep, which is known as "smoothness of falling asleep", and this can easily be quantitatively assessed by measuring an amount of time required for falling asleep (hereinafter, referred as "sleep latency"), requiring information on how long the user requires to actually fall asleep from the time the user gets into the bed, i.e., the time difference between the time at which the user intends to fall asleep and the time at which the user actually falls asleep. In accordance with a prior art disclosed in, for example, Japanese Patent Laid-open Application No. H5-245148, the time at which the user went to bed is used as the time at which the user intends to sleep. However, since the user may read books or watch TV after he/she went into the bed in many instances, it is often improper to regard the time at which the user went into the bed as the time at which the user intends to fall asleep, and the sleep latency calculated therefrom may be inaccurate.

For the two applications described above, it is required to provide means for easily and accurately detecting the time at which the user intends to fall asleep. In this regard, the invention and the examples described herein have made use of the fact that an alarm clock is set or a light is turned off or down usually right before going to sleep. That is, by approximating the time at which the user sets the alarm clock or turns the light off or down below a specific illuminance as the time at which the user intends to fall asleep, it is possible to more accurately determine the sleep latency. Further, by setting the time at which the lighting is converted from the normal illumination to the melatonin illumination two hours prior to the time at which the user sets the alarm clock or turned the light off or down below the specific illuminance, it is possible to more accurately control the time of lighting conversion from the normal illumination to the melatonin illumination.

Further, it is also known that the body movement is reduced when the sleep quality is improved whereas the body movement is increased when the sleep quality is deteriorated, and thus the quality of sleep can be evaluated based on the body movement during the sleep. Accordingly, there is proposed a device for measuring the body movement to evaluate the user's sleep quality (see, for example, Japanese patent Laid-open Application No. 2002-315738).

However, in accordance with the above-mentioned device for measuring the body movement, although the evaluation of the user's sleep quality is performed, it is not precise enough to evaluate a depth of sleep varying with time. To solve this problem, there is also proposed a method for evaluating the user's sleep quality more specifically by additionally obtaining information on the user's heartbeat and the like to perform the evaluation based thereon as well as the body movement. In accordance with this method, although a preciseness of the sleep evaluation can be enhanced, one or more sensors contacting the user's body are needed to measure the user's heartbeat and the like, which causes inconveneinces to the user or makes its system complicated and too expensive.

In this regard, the inventors noticed that precise information about the user's sleep could be obtained based only on the body movement by employing a technique of gradually intensifying the light before wake-up. In accordance with the technique of gradually intensifying (the) light before wake-up, which is originally developed as a therapy of seasonal affective disorder like winter depression, a sleeper is exposed to a gradually intensifying light from certain time before the wake-up time, thereby inducing an adjustment of the sleeper's biological rhythm. It is reported that a comfortable awakening can be induced by the light gradually intensifying until the wake-up time, and that, although the sleeper's eyes are closed during sleeping, the light is transmitted through the sleeper's eyelids and thus activates a part of brain to lead to awakening (see Noguchi, H., Shirakawa, S., Komada, Y., Koyama, E., and Sakaguchi, T., "Improved Quality of Awakening by Simulating Dawn Lighting with an Ordinary Ceiling Light", Journal of Illuminating Engineering Institute of Japan, 2001, Vol. 85, No. 5, pp. 315-322). Further, there is developed a bedroom lighting device capable of gradually increasing a light intensity. In addition, there are also disclosed an illumination device capable of gradually increasing a light intensity, and a awakening system having a body movement measuring unit for measuring the user's body movement (see, for example, Japanese Patent Laid-open Application No. H5-337103).

Although the above-mentioned technique of gradually intensifying the light can induce a comfortable awakening similar to that induced at daybreak, it has not yet been applied to the sleep evaluation. However, it can be predicted that a sleeper will show a body movement in response to the awakening effect of the gradually intensifying light more frequently if he/she has a healthy and sufficient sleep, because a deep sleep occurs more frequently during an earlier stage of the sleep and a shallow sleep occurs more frequently during a later stage of the sleep. On the other hand, if an amount of the sleep is not sufficient or the sleep quality is not good, the deep sleep may occur during the later stage of the sleep at a frequency higher than that of the above case, and therefore the sleeper will show a less body movement in response to the awakening effect of the gradually intensifying light. The present invention, developed by applying the above-mentioned principle, can evaluate the sleep quality based only on the body movement by measuring the reactive body movement in response to the gradually intensifying the light before wake-up.

### Disclosure of Invention

It is, therefore, an object of the present invention to provide an illumination system for detecting a time at which a user intends to fall asleep based on a time at which the user set an alarm clock or turned off a light just before sleeping, allowing the sleep quality to be precisely evaluated and to be improved by controlling an illumination for reducing melatonin suppression to be controlled.

It is another object of the present disclosure to provide an illumination system capable of evaluating a sleep quality based only on a body movement by measuring a reactive body movement in response to a gradually intensifying light before wake-up in addition to measuring a body movement during sleeping for evaluating a smoothness of falling asleep and/or a sleep state, thereby making it possible to detect a deficiency of sleep amount and a deterioration in the sleep quality. According to the present invention, there is provided an illumination system including: a lighting device; a manipulation device for a user to manipulate when the user intends to fall asleep; a control unit adapted to detect a manipulation time at which the manipulation device is manipulated; and a memory unit adapted to store a reference time, wherein the control unit is adapted to start an illumination control to reduce the user's melatonin suppression if a current time is a preset timing before the reference time stored in the memory, wherein, if the current time is not yet the preset timing, the control unit determines whether the manipulation device is manipulated and starts the illumination control from the manipulation time if it is determined that the manipulation device is manipulated, wherein by the illumination control, an intensity of a light from the lighting device is gradually or stepwisely reduced during the preset timing, and wherein when the manipulation device is manipulated, the control unit updates the reference time based on the manipulation time and stores the updated reference time in the memory unit. In this manner, a conversion from a normal illumination to an illumination for reducing the user's melatonin suppression can be automatically performed according to the user's daily routine based on the start time of the sleep latency.

Preferably, the illumination system further includes a body movement measuring unit for detecting whether the user falls asleep, wherein the control unit further includes an evaluation subunit for evaluating the user's sleep quality based on a detection result of the body movement measuring unit and the start time of the sleep latency.

In this manner, the sleep latency calculation and the sleep evaluation can be performed precisely with a simple configuration, thereby helping the user's health management.

Preferably, the manipulation device is an alarm clock or a switch for manipulating the lighting device.

In this manner, the start time of the sleep latency can be detected in a cost-effective manner with a simple configuration.

Preferably, the reference time is the start time of measuring the sleep latency.

In accordance with another aspect of the present disclosure there is provided an illumination system including: a lighting device capable of illuminating gradually intensifying light; a body movement measuring unit for measuring a user's body movement; and an evaluation unit for evaluating the user's sleep quality based on an illumination of the gradually intensifying light and the user's body movement measured by the body movement measuring unit from a time at which the user goes to bed to a time at which the user wakes up via a time at which the user falls asleep.

In this manner, by measuring the reactive body movement in response to the gradually intensifying light before wake-up, the quality of awakening can be evaluated based on the depth of sleep near the wake-up time.

Preferably, the evaluation unit evaluates the use's sleep quality based on an evaluation result of a smoothness of falling asleep obtained based on a time period from a time at which the user goes to bed and a time at which the user's body movement is reduced below a specific level, a sleep maintenance obtained based on the user's body movement measured during sleeping, and a quality of awakening obtained based on the user's body movement in response to the gradually intensifying light before wake-up. Thus, the sleep quality is evaluated based on the user's body movement in view of the smoothness of falling asleep, the sleep maintenance and the quality of awakening.

In this manner, it is possible to perform a more precise sleep evaluation including a detection of a sleep deficiency or a fatigue accumulation based on the smoothness of falling asleep obtained from the user's body movement, the sleep maintenance, and the quality of awakening obtained from the user's reactive body movement in response to the gradually intensifying light.

Preferably, the body movement measuring unit is an infrared sensor that does not physically contact the user during sleeping.

In this manner, it is possible to prevent an unpleasant feeling experienced during sleeping in case of using a sensor physically contacting the user.

In accordance with still another aspect of the present disclosure there is provided a sleep management system including: the illumination system described above; a data storage unit for storing an evaluation result of the sleep quality; a communications unit for sending the evaluation result of the sleep quality to a terminal device via a communications channel; and a data processing unit capable of communicating with the terminal device..

In this manner, since the result of the sleep evaluation does not need to be stored in a memory of the illumination system, the illumination system does not need a memory included therein. Further, a sleep state of the user can be checked by using the terminal device regardless of time and place. In addition, the result of the sleep evaluation can be sent to a medical expert such as a medical institution via the data processing unit by using the terminal device, and the user can easily receive an expert's advice or statistic data (for example, sleeping times of other people of a same age as the user) based on the result of the sleep evaluation.

### Brief Description of Drawings

The above and other objects and features of the present invention will become apparent from the following description of preferred embodiments, given in conjunction with the accompanying drawings, in which:
Fig. 1 shows a configuration of an illumination system capable of reducing melatonin suppression in accordance with a first embodiment of the present invention;
Fig. 2 represents a timing diagram describing an operation of the illumination system in accordance with the first embodiment of the present invention;
Fig. 3 depicts a flow chart describing an operation of the illumination system in accordance with the first embodiment of the present invention;
Fig. 4 presents a configuration of an illumination system in accordance with a first example
Fig. 5 represents a timing diagram describing a sleep evaluation performed by a control unit in the illumination system in accordance with the first example
Figs. 6A and 6B offer flow charts of the sleep evaluation performed by the illumination system in accordance with the first example
Fig. 7 describes an example of the sleep evaluation performed by the illumination system in accordance with the first example and
Fig. 8 shows a sleep management system including an illumination system in accordance with a second example.

### Best Mode for Carrying Out the Invention

Hereinafter, illumination systems in accordance with preferred embodiments of the present invention will be described with reference to accompanying drawings.

### (First Embodiment)

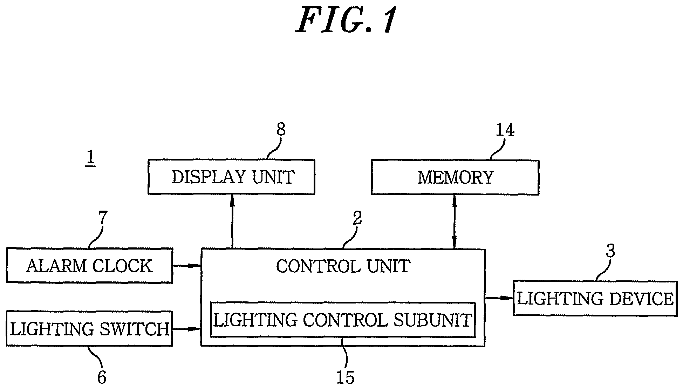

Fig. 1 shows a configuration of an illumination system capable of reducing melatonin suppression in accordance with a first embodiment of the present invention. An illumination system 1 includes a control unit 2 for controlling the illumination; a lighting device 3 controlled by the control unit 2 to perform a conversion from the normal illumination to the melatonin illumination; a lighting switch 6 for turning on, off or down the lighting device 3 by a manipulation of a user; an alarm clock 7; a display unit 8; and a memory 14 for storing a time at which the user intends to fall asleep, and a reference time used for computing a time for starting the melatonin illumination. The control unit 2 includes a lighting control subunit 15 for controlling a conversion from the normal illumination to the melatonin illumination, wherein the lighting control subunit 15 is preferably a software installed in the control unit 2. The melatonin illumination has an illuminance range, for example, below 100 lux including a complete darkness, or same as that described in Japanese Patent Application No. 2005-074796.

The lighting switch 6 and the alarm clock 7 are manipulation devices for a user to manipulate when the user intends to fall asleep for indicating a time at which the user intends to fall asleep, i.e., a sleep intention time, and this is equivalent to the time at which the user turn the lighting switch 6 off or down below a specific illuminance or set the alarm clock 7 right before sleeping. At the sleep intention time, the control unit 2 obtains an updated reference time based on thus obtained sleep intention time and a reference time stored in the memory 14. Thereafter, the control unit 2 stores the updated reference time as the reference time in the memory 14, and sets a subsequent conversion time for performing a conversion from the normal illumination to the melatonin illumination to be preset hour(s), e.g., two hours, before the reference time that has been just updated. A specific method of obtaining the reference time from the sleep intention time and a previous reference time stored in the memory 14 will be described later.

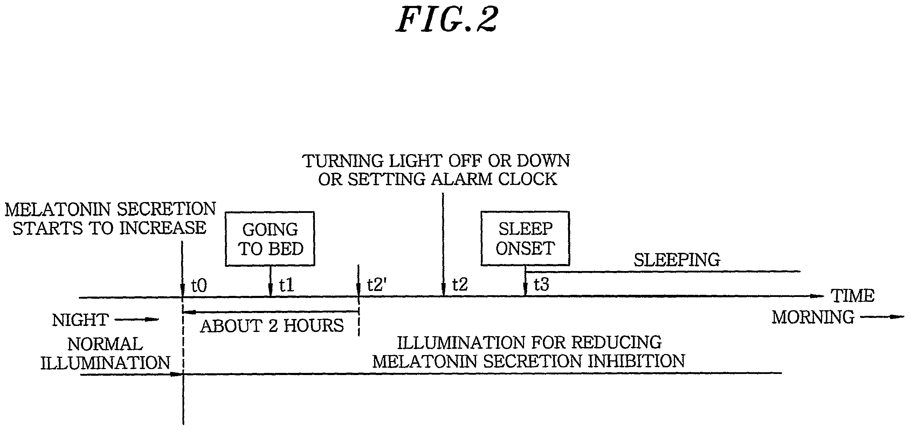

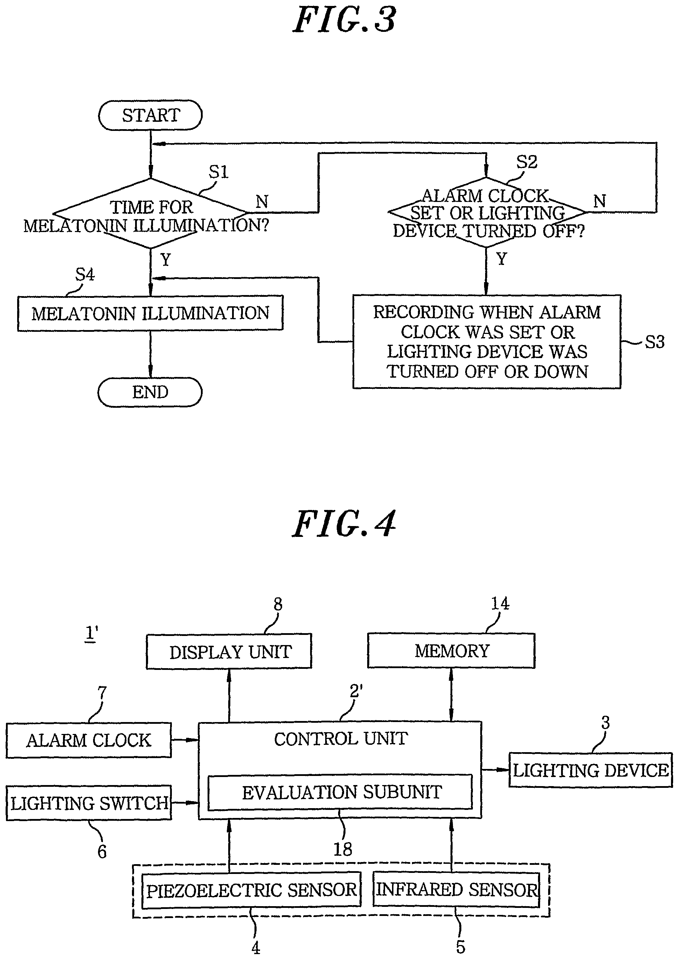

Figs. 2 and 3 represent a timing diagram and a flow chart describing an operation of the illumination system 1 in accordance with the first embodiment of the present invention, respectively. In Fig. 2, t1 is a time at which the user went to bed (hereinafter, abbreviated as "going-to -bed time"), t2' is the reference time, t2 is the sleep intention time, and t3 is a time at which the user fell asleep (hereinafter, abbreviated as "sleep onset time"), wherein a sequence of events is arranged according to an ordinary daily routine. As shown therein, the secretion of melatonin starts to increase from about two hours before a time at which the user normally intends to fall asleep. Accordingly, the control unit 2 performs a conversion from the normal illumination to the melatonin illumination at a conversion time t0 that is about two hours before the reference time t2' stored in the memory 14. Melatonin is known to promote sleeping, and a sleep quality is influenced by an amount of melatonin secretion. In this regard, the illumination system 1 controls the lighting to promote the melatonin secretion.

As shown in Fig. 3, the control unit 2 checks whether or not the current time is the conversion time t0 (step S1), and if the result is YES, the melatonin illumination is performed (step S4). In this melatonin illumination, the light has an illuminance below, e.g., 100 lux, or same as that described in Japanese Patent Application No. 2005-074796, and preferably, becomes turned off in a gradual or stepwise manner during a specific period of time, e.g., two hours. Further, if the result of step S1 is NO, the control unit 2 checks whether or not the alarm clock 7 has been set or the lighting switch 6 has been turned off or down below the specific illuminance (step S2), and if the result is YES, the control unit 2 stores the timing of the manipulation as the sleep intention time t2 in the memory 14 (step S3). Thereafter, the melatonin illumination is performed (step S4). Preferably, the light is maintained to be off if the lighting switch 6 is detected to be turned off at step S2, and preferably, the light is turned off in a gradual or stepwise manner during a specific period of time, e.g., two hours if the lighting switch 6 is detected to be turned down below the specific illuminance at step S2. The control of the illumination described above is performed based on the sleep intention time t2 stored in the memory 14. As described above, in accordance with the illumination system 1 in the first embodiment, the conversion from the normal illumination to the melatonin illumination can be carried out automatically based on the user's daily routine, so that the user does not need to convert the illumination manually. The controller 2 may be programmed such that the reference time is set to be equal to the sleep intention time stored in the memory 14, and, in this case, the memory 14 may store only the sleep intention time. Alternatively, the controller 2 may be programmed such that the reference time is set to be an average of sleep intention times of the previous week. Alternatively, the reference time can be obtained based on the user's work schedule if the wakeup time varies as in case of, e.g., a three-shift system. In this case, the illumination system 1 further includes an input unit for the setting.

### (First Example)

In accordance with a first example there is provided an illumination system capable of evaluating a sleep quality by assessing the smoothness of falling asleep, the sleep maintenance and the quality of awakening.

Firstly, the smoothness of falling asleep can be quantitatively assessed by measuring a sleep latency, i.e., an amount of time required for falling asleep. Specifically, a sleep intention time, i.e., a time at which a user intends to fall asleep, is set by detecting the manipulation of an alarm clock or an illumination switch. Further, the sleep onset time is obtained by measuring the user's body movement and determining a time at which the body movement is reduced below a specific level to be the sleep onset time. Finally, the sleep latency is calculated by obtaining a time difference between the sleep intention time and the sleep onset time, and thus calculated sleep latency is used as an index of the smoothness of falling asleep.

Secondly, the sleep maintenance can be assessed using the body movement during sleeping, because the body movement increases if the user becomes awake in the middle of the sleep or a shallow sleep occurs more frequently.

Thirdly, the quality of awakening can be quantitatively assessed by measuring the reactive body movement in response to the gradually intensifying light before wake-up. If the amount of sleep is sufficient and the sleep quality is good, a relatively shallow sleep occurs before the wake-up time. Therefore, in this case, the user will show more reactive body movement in response to the gradually intensifying light before wake-up, and the awakening can be evaluated as healthy and comfortable. However, if the amount of sleep is insufficient and the sleep quality is not good, a deep sleep may occur before the wake-up time more frequently than that of the above case. Therefore, in this case, the user will show less reactive body movement in response to the gradually intensifying light before wake-up, and the awakening will be uncomfortable and physiologically unhealthy. In this manner, the quality of awakening can be quantitatively assessed using a gradually intensifying light and measuring the reactive body movement in response thereto.

Information about the sleep can be obtained respectively from the three sleep evaluation factors described above. However, by considering all of them at the same time, the information about the sleep becomes more precise, and, most importantly, a sleep deficiency or a fatigue accumulation can be detected. If the user's desire for sleep is intensified by a sleep deficiency or a fatigue accumulation, but an amount of sleeping time is not sufficient for satisfying the intensified desire for sleep, the deep sleep may occur before the wake-up time more frequently as described above. Therefore, in this case, the user will show a less reactive body movement in response to the gradually intensifying light.

The above-described reduction of the reactive body movement in response to the gradually intensifying light before wake-up is caused by the deep sleep before the wake-up time in case the user has problems with a smoothness of falling asleep or a sleep quality. Therefore, it needs to be verified that the user does not have these problems in order to detect that the user has a sleep deficiency or fatigue caused thereby. Accordingly, by synthetically combining the smoothness of falling asleep, the sleep maintenance and the quality of awakening, a sleep deficiency or fatigue caused thereby can be detected more easily. In other words, if the smoothness of falling asleep and the sleep maintenance is evaluated as good, but the quality of awakening is not good, it can be inferred that the amount of sleeping time is not sufficient for satisfying the desire for sleep, and the user can be informed that he/she may have a sleep deficiency or fatigue caused thereby.

Hereinafter, the illumination system in accordance with the example will be described with reference to Figs. 4 to 7.

Fig. 4 illustrates a configuration of the illumination system 1' in accordance with the example. As shown therein, the illumination system 1' includes a control unit 2' for controlling the illumination and the system; a lighting device 3 controlled by the control unit 2' and capable of gradually intensifying the light; a piezoelectric sensor 4 or an infrared sensor 5 functioning as a body movement measuring unit for measuring the body movement of the user during sleeping; a lighting switch 6 for turning on or off the lighting device 3 in response to the user's manipulation; an alarm clock 7 for setting a wake-up time; a display unit 8 made of, e.g., a liquid crystal display; and a memory 14 for storing information such as the sleep intention time.

In the example, the control unit 2' includes an evaluation subunit 18 for performing the sleep evaluation by using the gradually intensifying light of the lighting device 3 and the body movement from the sleep intention time to the wake-up time measured by the body movement measuring unit. Preferably, the evaluation subunit 18 is a software installed in the control unit 2'. The sleep evaluation is performed based on the smoothness of falling asleep, the sleep maintenance and the quality of awakening.

Preferably, the piezoelectric sensor 4 is installed in the user's bed. Further, the infrared sensor 5 may be formed as a single body with the lighting device 3 or as a separate device distinguished from the lighting device 3. The piezoelectric sensor 4 or the infrared sensor 5, measures the body movement of the user during sleeping, and detects a sleep onset time by determining a time at which the body movement is reduced below a specific level to be the sleep onset time. As the body movement measuring unit, either the piezoelectric sensor 4 or the infrared sensor 5 can be used. The lighting switch 6 and the alarm clock may be configured as a single body.

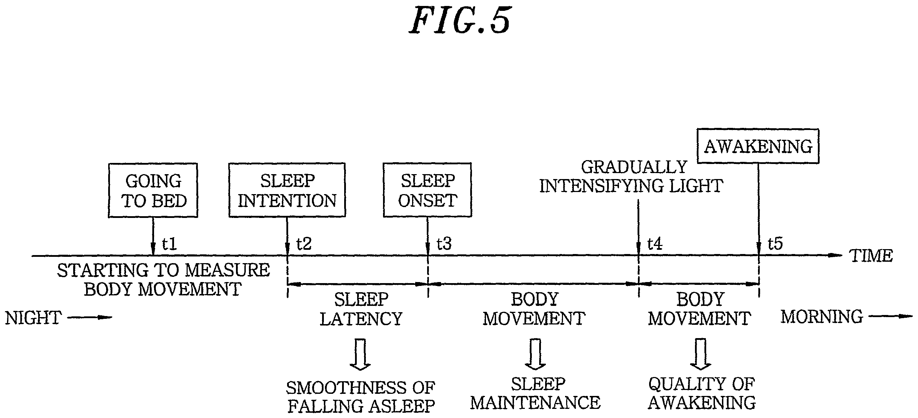

Fig. 5 represents a timing diagram describing the sleep evaluation performed by the control unit 2' in the illumination system 1' in accordance with the example wherein a sequence of events is arranged according to an ordinary daily routine. The control unit 2' detects the going-to-bed time t1 by detecting a first body movement greater than a specific level; the sleep intention time t2 by detecting a time at which the alarm clock is set or the lighting switch 6 is turned off or down below a specific illuminance; and the sleep onset time t3 by detecting a reduction in the body movement below a specific level; and the sleep latency by calculating a time difference between the sleep intention time t2 and the sleep onset time t3. Further, the control unit 2' evaluates the sleep maintenance based on the body movement measured from the sleep onset time t3 to the wake-up time t5. In addition, the control unit 2' performs a gradually intensifying illumination from a time t4 that is a preset hour(s) before the wake-up time t5, and measures the reactive body movement in response thereto to evaluate the quality of awakening based thereon. The evaluation results are displayed on the display unit 8 so that the evaluation results can be shown to the user after the wake-up.

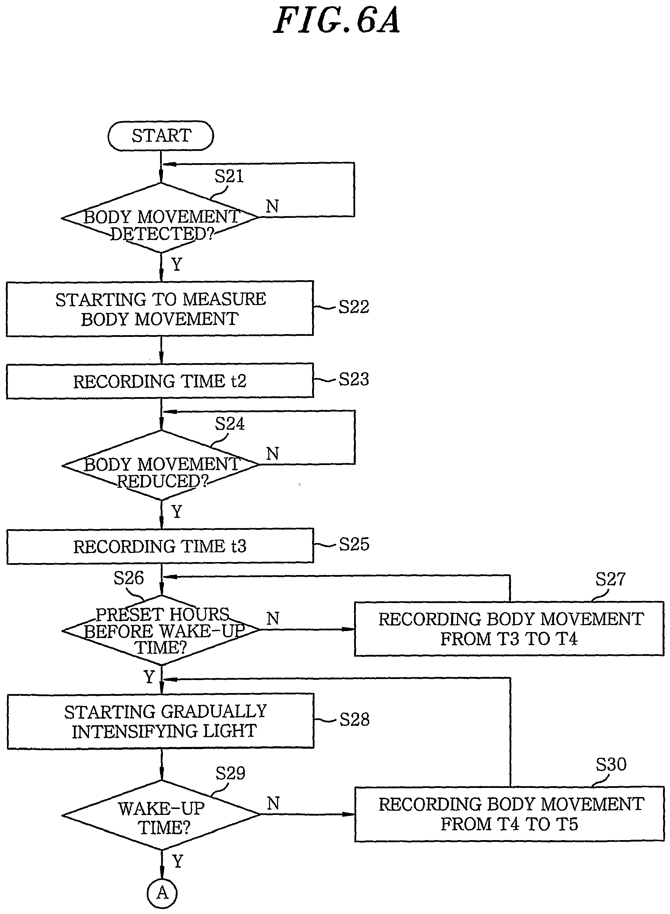

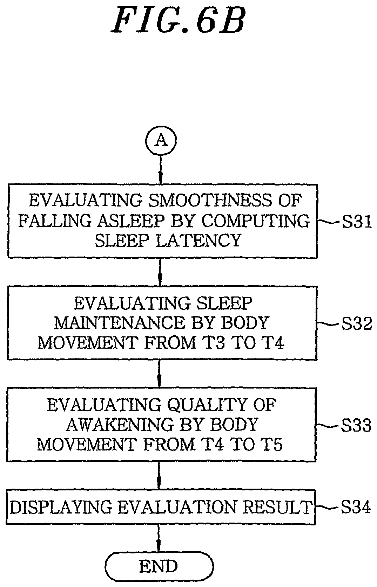

Figs. 6A and 6B depict flow charts of the sleep evaluation performed by the illumination system 1'. The control unit 2' checks whether or not the body movement is detected based on signals from the body movement measuring unit (step S21). If the result is YES, the control unit 2' starts to measure the body movement of the user (step S22). Then, if the alarm clock 7 is set or the lighting switch 6 is turned off or down below a specific illuminance, the control unit 2' stores the timing of this manipulation of the alarm clock 7 or the lighting switch 6 as the sleep intention time t2 in the memory 14 (step S23). Thereafter, the control unit 2' checks whether or not the body movement is reduced below the specific level (step S24), and if the result is YES, stores the timing of the reduction in the body movement as the sleep onset time t3 (step S25). Then, the control unit 2' checks whether or not the current time is a preset hour(s) before the wake-up time of the alarm clock (step S26), and if the result is NO, records the body movement from the sleep onset time t3 to the time t4 shown in Fig. 5 (step S27). However, if the result of step S26 is YES, the control unit 2' starts the gradually intensifying illumination (step S28). Then, the control unit 2' checks whether or not the current time is the wake-up time of the alarm clock 7 (step S29), and if the result is NO, the control unit 2' records the body movement from the time t4 to the wake-up time t5 (step S30). However, if the result of step S29 is YES, the control unit 2' calculates the sleep latency by obtaining a time difference between the sleep intention time t2 and the sleep onset time t3. Then, the control unit 2' evaluates the smoothness of falling asleep based on the sleep latency (step S31); the sleep maintenance based on the record of the body movement from the sleep onset time t3 to the time t4 shown in Fig. 5 (step S32); and the quality of awakening based on the record of the body movement from the time t4 to the wake-up time t5 (step S33). Thereafter, the control unit 2' has the evaluation results displayed on the display unit 8 (step S34).

In Figs. 6A and 6B, it is assumed that the user goes to bed before the lighting switch 6 or the alarm clock 7 is manipulated. However, the control unit 2' may be configured such that, if the user goes to bed after the lighting switch 6 or the alarm clock 7 is manipulated, the control unit 2' detects the time at which the user went to bed by detecting the body movement, and stores the detected timing as the sleep intention time t2 in the memory 14.

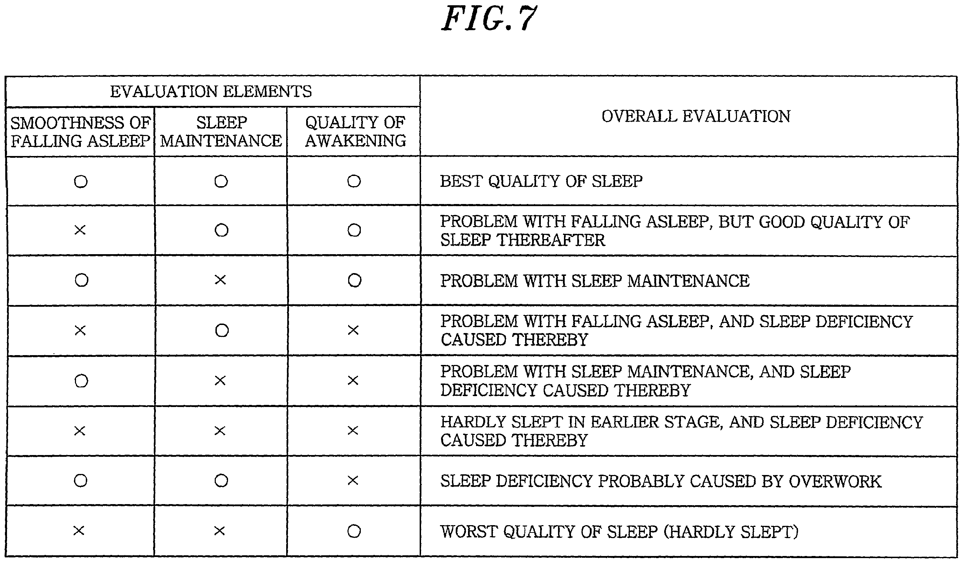

As described above, the illumination system 1' can evaluate the smoothness of falling asleep and the sleep maintenance based on the body movement measured during sleeping, and the quality of awakening based on the reactive body movement in response to the gradually intensifying light before wake-up. Further, by combining the above-mentioned results, the illumination system 1' can perform a more precise sleep evaluation such as a detection of a sleep deficiency or fatigue accumulation caused thereby. Fig. 7 offers an example of the sleep evaluation. In Fig. 7, O and X respectively represent a good evaluation and a bad evaluation on each of the evaluation factor, and the synthetic evaluation is obtained by combining them.

### (Second Example)

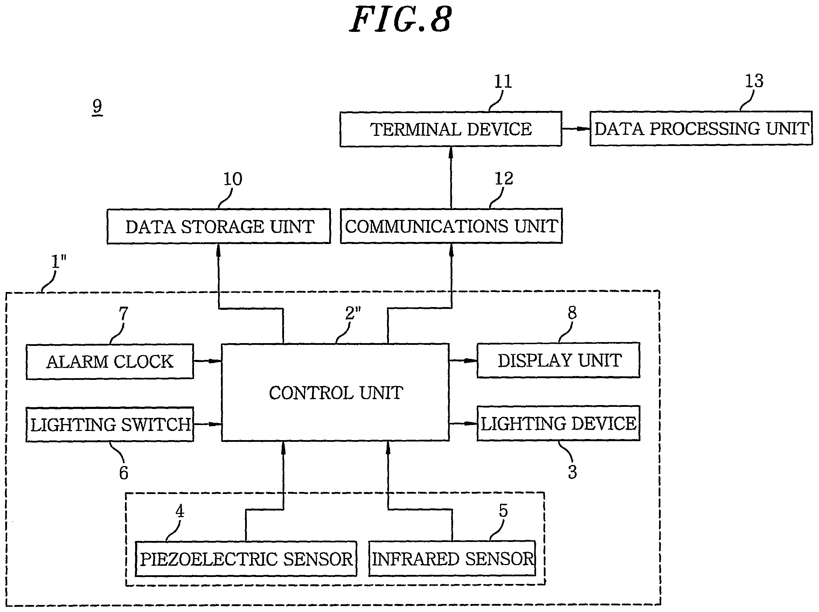

Fig. 8 represents a sleep management system 9 including an illumination system 1" wherein the illumination system 1'' is same as the illumination system 1' except that the illumination system 1'' does not include the memory 14. The sleep management system 9 includes the illumination system 1"; a data storage unit 10 for storing a result of a sleep evaluation; a communications unit for sending the result of the sleep evaluation to a terminal device 11 via a communications channel; and a data processing unit 13 capable of communicating with the terminal device 11 via, for example, the Internet. The data storage unit 10, configured by an all-purpose memory device, does not need to be embedded in the illumination system 1". Preferably, the data storage unit 10 is connected on-line to the illumination system 1''. The terminal device 11 is, for example, a mobile phone, a PDA (Personal Digital Assistant) or a facsimile. The communications unit 12 is an interface for exchanging data with the illumination system 1" by wired or infrared communications. The data processing unit 13 is an external server such as a WEB server capable of accessing the illumination system 1'' by using the terminal device 11.

With this configuration, since the result of the sleep evaluation does not need to be stored in a memory in the illumination system, the illumination system 1" does not need a memory included therein. Further, a sleep state of the user can be checked by using the terminal device 11 regardless of time and place. In addition, the result of the sleep evaluation can be sent to a medical expert such as a medical institution via the data processing unit 13 by using the terminal device 11, and the user can easily receive an expert's advice or statistic data (for example, sleeping times of other people of a same age as the user) based on the result of the sleep evaluation.

Further, the examples are not limited to the configurations described above, but may be modified without departing from the scope of the disclosure. For example, other means than described above may be used as the body movement measuring unit, as long as it can measure the user's body movement. In addition, instead of the reactive body movement in response to the gradually intensifying light, it is possible to use other physiological index such as a brain wave or a heartbeat in response to the gradually intensifying light. Furthermore, the smoothness of falling asleep and the sleep maintenance may also be evaluated by detecting the brain wave, the heartbeat and/or the blood pressure of the user instead of the body movement of the user. In this case, the illumination system 1'' includes a device for detecting the brain wave, the heartbeat and/or the blood pressure in lieu of the piezoelectric sensor 4 or the infrared sensor 5.

Further, the illumination system 1 in accordance with the embodiment may further include the piezoelectric sensor 4 or the infrared sensor 5, and the control unit 2 in accordance with the embodiment may further include the evaluation subunit 18. In this case, the illumination system of this configuration can not only reduce the melatonin suppression but also evaluate the user's sleep quality by, for example, calculating the sleep latency, wherein the method of calculating the sleep latency and evaluating the user's sleep quality based thereon is same as that described in the first examples.

Further, in this case, it is preferable that the illumination is controlled according to the user's sleep quality. Since the user falls asleep rapidly if melatonin is secreted sufficiently at night, it can be estimated that the user's melatonin secretion is not sufficient if the sleep latency is long. Therefore, if the sleep latency is calculated to be long, it is preferable that the conversion time (t0 shown in Fig. 2) of the next night is set to be earlier so that the melatonin secretion can be increased.

Still further, although the illumination systems have been described to include the alarm clock the illumination system may be configured such that it does not include the alarm clock.

While the invention has been shown and described with respect to the preferred embodiments, it will be understood by those skilled in the art that various changes and modification may be made without departing from the scope of the invention as defined in the following claims.

## Claims

1. An illumination system (1) comprising:
a lighting device (3);
a manipulation device (6, 7) for a user to manipulate when the user intends to fall asleep;
a control unit (2) adapted to detect a manipulation time at which the manipulation device (6, 7) is manipulated; and
a memory unit (14) adapted to store a reference time,
wherein the control unit (2) is adapted to start an illumination control to reduce the user's melatonin suppression if a current time is a preset timing before the reference time stored in the memory (14),
wherein, if the current time is not yet the preset timing, the control unit (2) determines whether the manipulation device (6, 7) is manipulated and starts the illumination control from the manipulation time if it is determined that the manipulation device (6, 7) is manipulated,
wherein by the illumination control, an intensity of a light from the lighting device (3) is gradually or stepwisely reduced during the preset timing, and
wherein when the manipulation device (6, 7) is manipulated, the control unit (2) updates the reference time based on the manipulation time and stores the updated reference time in the memory unit (14).

2. The illumination system (1) of claim 1, wherein the manipulation device (6, 7) is an alarm clock (7) or a switch (6) for manipulating the lighting device (3).

3. The illumination system (1) of claim 1, wherein the reference time is a latest previous manipulation time.

4. The illumination system (1) of claim 1, wherein the preset timing is 2 hours before the reference time stored in the memory (14).

## Patentansprüche

1. Beleuchtungssystem (1) umfassend:
eine Beleuchtungsvorrichtung (3);
eine Manipulationsvorrichtung (6, 7) für einen Benutzer zum Manipulieren, wenn der Benutzer beabsichtigt, einzuschlafen;
eine Steuereinheit (2), die ausgestaltet ist, eine Manipulationszeit zu erfassen, bei der die Manipulationsvorrichtung (6, 7) manipuliert wird; und
eine Speichereinheit (14), die ausgestaltet ist, eine Bezugszeit zu speichern,
wobei die Steuereinheit (2) ausgestaltet ist, eine Beleuchtungssteuerung zu starten, um die Melatoninunterdrückung des Nutzers zu reduzieren, wenn eine aktuelle Zeit ein voreingestelltes Timing vor der Bezugszeit ist, die in dem Speicher (14) gespeichert ist,
wobei die Steuereinheit (2) bestimmt, wenn die aktuelle Zeit noch nicht das voreingestellte Timing ist, ob die Manipulationsvorrichtung (6, 7) manipuliert wird und die Beleuchtungssteuerung von der Manipulationszeit startet, wenn bestimmt wird, dass die Manipulationsvorrichtung (6, 7) manipuliert wird,
wobei durch die Beleuchtungssteuerung eine Intensität eines Lichts von der Beleuchtungsvorrichtung (3) allmählich oder schrittweise während des voreingestellten Timings reduziert wird, und
wobei die Steuereinheit (2), wenn die Manipulationsvorrichtung (6, 7) manipuliert wird, die Bezugszeit basierend auf der Manipulationszeit aktualisiert und die aktualisierte Bezugszeit in der Speichereinheit (14) speichert.

2. Beleuchtungssystem (1) nach Anspruch 1, bei dem die Manipulationsvorrichtung (6, 7) ein Wecker (7) oder eine Schaltung (6) zum Manipulieren der Beleuchtungsvorrichtung (3) ist.

3. Beleuchtungssystem (1) nach Anspruch 1, bei dem die Bezugszeit eine späteste bisherige Manipulationszeit ist.

4. Beleuchtungssystem (1) nach Anspruch 1, bei dem das voreingestellte Timing 2 Stunden vor der Bezugszeit ist, die in dem Speicher (14) gespeichert ist.

## Revendications

1. Système d'illumination (1) comprenant :
un dispositif d'éclairage (3) ;
un dispositif de manipulation (6, 7) à manipuler par un utilisateur lorsque l'utilisateur a l'intention de s'endormir ;
une unité de commande (2) adaptée pour détecter un temps de manipulation auquel le dispositif de manipulation (6, 7) est manipulé ; et
une unité de mémoire (14) adaptée pour stocker un temps de référence,
dans lequel l'unité de commande (2) est adaptée pour démarrer une commande d'illumination pour réduire une suppression de mélatonine de l'utilisateur si un temps actuel est un moment préréglé avant le temps de référence stocké dans la mémoire (14),
dans lequel, si le temps actuel n'est pas encore le moment préréglé, l'unité de commande (2) détermine si le dispositif de manipulation (6, 7) est manipulé et démarre la commande d'illumination à partir du temps de manipulation si on détermine que le dispositif de manipulation (6, 7) est manipulé,
dans lequel, grâce à la commande d'illumination, une intensité d'une lumière provenant du dispositif d'éclairage (3) est réduite progressivement ou par paliers pendant le moment préréglé, et
dans lequel, lorsque le dispositif de manipulation (6, 7) est manipulé, l'unité de commande (2) met à jour le temps de référence d'après le temps de manipulation et stocke le temps de référence mis à jour dans l'unité de mémoire (14).

2. Système d'illumination (1) selon la revendication 1, dans lequel le dispositif de manipulation (6, 7) est un réveil (7) ou un interrupteur (6) pour manipuler le dispositif d'éclairage (3).

3. Système d'illumination (1) selon la revendication 1, dans lequel le temps de référence est un temps de manipulation précédent le plus récent.

4. Système d'illumination (1) selon la revendication 1, dans lequel le moment préétabli est 2 heures avant le temps de référence stocké dans la mémoire (14).
